# EUROPEAN PATENT APPLICATION

(11) **EP 4 417 654 A1**
(43) Date of publication of application: **21.08.2024**
(21) Application number: 24155356.9
(22) Date of filing: 01.02.2024
(51) Int. Cl.: C09C 1/48, C08K 3/04, C08L 21/00, G01N 21/94, G01N 33/44

(54) **METHOD FOR DISCRIMINATING CARBON BLACK**

(30) Priority: 17.02.2023 JP 2023023248
(71) Applicant: Sumitomo Rubber Industries, Ltd., Kobe-shi, Hyogo 651-0072 (JP)
(72) Inventor: OISHI, Kentaro, Kobe-shi, Hyogo, 651-0072 (JP); KAGEYUKI, Ikuo, Kobe-shi, Hyogo, 651-0072 (JP)
(74) Representative: Manitz Finsterwald Patent- und Rechtsanwaltspartnerschaft mbB

(57) **Abstract**

The present invention provides a method for discriminating carbon black which can discriminate whether or not a carbon black is a recovered carbon black. Provided is a method for discriminating whether or not a carbon black is a recovered carbon black, including examining the presence of an inorganic substance on or near a surface of the carbon black.

## Description

### TECHNICAL FIELD

The present invention relates to a method for discriminating carbon black.

### BACKGROUND ART

Lately, there is a demand for improving the recycling rate of tires from the standpoint of environmental protection, etc., and use of recovered carbon black is considered. Recovered carbon black can be obtained by, for example, thermal decomposition of used rubber products such as waste tires.

### SUMMARY OF INVENTION

### TECHNICAL PROBLEM

In the consideration of the use of recovered carbon black, it is desired to discriminate whether the carbon black is a recovered carbon black or a conventional general-purpose carbon black. However, conventional analysis methods such as measurement of primary particle size with an electron microscope and measurement of aggregate distribution based on DBP oil absorption, IA, CTAB, or particle size distribution have difficulty in discriminating between a recovered carbon black and a conventional carbon black.

The present invention aims to solve the problem and provide a method for discriminating carbon black which can discriminate whether or not a carbon black is a recovered carbon black.

### SOLUTION TO PROBLEM

The present invention relates to a method for discriminating whether or not a carbon black is a recovered carbon black, including
examining the presence of an inorganic substance on or near a surface of the carbon black.

### ADVANTAGEOUS EFFECTS OF INVENTION

The method for discriminating whether or not a carbon black is a recovered carbon black according to the present invention includes examining the presence of an inorganic substance on or near a surface of the carbon black. This method can discriminate whether or not a carbon black is a recovered carbon black.

### BRIEF DESCRIPTION OF DRAWINGS

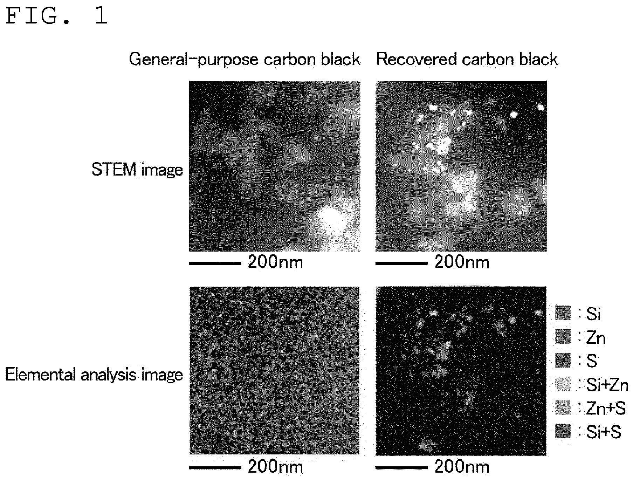

FIG. 1 shows STEM images of Blend 1 (elastomer composition containing a general-purpose carbon black) and Blend 2 (elastomer composition containing a recovered carbon black) captured in STEM mode with TEM and elemental analysis images thereof.

### DESCRIPTION OF EMBODIMENTS

The present invention relates to a method for discriminating whether or not a carbon black is a recovered carbon black. The method discriminates whether or not a carbon black is a recovered carbon black by examining the presence of an inorganic substance on or near the surface of the carbon black.

Recovered carbon black includes carbon black obtained by thermal decomposition of used rubber products such as waste tires. Tires contain inorganic substances (for example, ZnO, silica, ZnS) which are not thermally decomposed. In the process of producing recovered carbon black, the inorganic substances may enter recovered carbon black and may be adsorbed to the surfaces of the carbon black particles. This feature is not observed in conventional general-purpose carbon black (new carbon black). Thus, analysis focusing on inorganic substances on the surface of the carbon black can distinguish between a recovered carbon black and a general-purpose carbon black. When an inorganic substance is present on or near the surface of a carbon black, the carbon black can be discriminated as a recovered carbon black, while when not, the carbon black can be discriminated as a general-purpose carbon black.

The discrimination method can discriminate whether a sample of a carbon black alone is a recovered carbon black or a general-purpose carbon black (new carbon black). The method also can discriminate whether a carbon black in an elastomer composition containing an elastomer component and the carbon black is a recovered carbon black or a general-purpose carbon black. The recovered carbon black and the general-purpose carbon black may each be one or two or more.

Examples of recovered carbon black include recovered carbon black produced through thermal decomposition of waste tires. The thermal decomposition of waste tires may be performed by known methods. Examples include thermal decomposition methods performed at a temperature of 650°C or higher.

Recovered carbon black usually has an average primary particle size of 10 to 100 nm. The lower limit is preferably 12 nm or more, more preferably 15 nm or more, while the upper limit is preferably 90 nm or less, more preferably 80 nm or less.

The average primary particle size of a carbon black can be determined by averaging the primary particle sizes of not less than 400 primary particles of the carbon black observed in the field of view of a transmissive or scanning electron microscope.

Commercially available recovered carbon black is usable. Examples include trade name PB365 available from Enrestec. PB365 is a recovered carbon black produced through thermal decomposition of waste tires and has a N₂SA of 76 m²/g. PB365 has an ash content of about 17% by mass.

Non-limiting examples of general-purpose carbon black which is not recovered carbon black include SAF, ISAF, HAF, FF, FEF, and GPF. Usable commercial products are available from Asahi Carbon Co., Ltd., Cabot Japan K. K., Tokai Carbon Co., Ltd., Mitsubishi Chemical Corporation, Lion Corporation, NSCC Carbon Co., Ltd, Columbia Carbon Co., Ltd., etc. These may be used alone or in combinations of two or more.

The discrimination method discriminates whether or not a carbon black is a recovered carbon black by examining the presence of an inorganic substance on or near the surface of the carbon black. When an inorganic substance is present on or near the surface of the carbon black, the carbon black can be discriminated as a recovered carbon black.

In the discrimination method, the phrase "on or near a surface of the carbon black" refers to, for example, on the surfaces of the particles of the carbon black or a region within a predetermined range from the surfaces of the particles. Examination of the presence (attaching, contacting, etc.) of an inorganic substance on the surfaces of the particles or in the region can discriminate whether or not the carbon black is a recovered carbon black.

In the discrimination method, the region on or near the surface of the carbon black is desirably a region from the surface of the carbon black to the height equal to 200 of the primary particle size of the carbon black. As described above, carbon black usually has an average primary particle size of 10 to 100 nm. Thus, whether the carbon black is a recovered carbon black or a general-purpose carbon black tends to be examined with high accuracy by examining the presence of an inorganic substance in a region from the surface to 20% as described above, specifically in a region from the surface to a height of 2 to 20 nm.

Non-limiting examples of the inorganic substance include known agents used in elastomer compositions, such as inorganic fillers, zinc oxide (ZnO), zinc sulfide (ZnS), and sulfur. The inorganic fillers may be materials known in the rubber field, such as silica, calcium carbonate, talc, alumina, clay, aluminum hydroxide, aluminum oxide, and mica.

The inorganic substance is desirably zinc (Zn). Whether the carbon black is a recovered carbon black or a general-purpose carbon black tends to be examined with high accuracy by examining the presence of zinc on or near the surface of the carbon black.

In the discrimination method, the presence of an inorganic substance on or near the surface of the carbon black can be examined using an electron microscope, for example. Specifically, the discrimination of whether a carbon black is a recovered carbon black or a general-purpose carbon black can be performed by conducting elemental analysis using an electron microscope image of the carbon black and examining the presence of an inorganic substance on or near the surface of the carbon black. In this case, the discrimination can be performed with high accuracy.

Any electron microscope is usable, including a scanning electron microscope (SEM) and a transmission electron microscope (TEM). In view of spatial resolution, TEM is desirable.

As described above, the discrimination method can discriminate whether or not a carbon black in an elastomer composition containing an elastomer component and the carbon black is a recovered carbon black or a general-purpose carbon black. In this case, the above technique can be used to discriminate whether or not the carbon black is a recovered carbon black. In this technique, the "on or near a surface of the carbon black", "inorganic substance", and "electron microscope" are also as described above.

Non-limiting examples of elastomer components usable in the elastomer composition include diene-based rubbers. Examples of diene-based rubbers include isoprene-based rubbers, polybutadiene rubber (BR), styrene-butadiene rubber (SBR), styrene-isoprene-butadiene rubber (SIBR), ethylene-propylene-diene rubber (EPDM), chloroprene rubber (CR), and acrylonitrile-butadiene rubber (NBR). Examples also include butyl-based rubbers and fluororubber. These may be used alone or in combinations of two or more.

Examples of the carbon black in the elastomer composition include the recovered carbon black and general-purpose carbon black. The recovered carbon black and the general-purpose carbon black may each be one or two or more.

The amount of the carbon black (total amount of recovered carbon black and other carbon black) in the elastomer composition is not limited. For example, the amount per 100 parts by mass of the elastomer component is 1 to 150 parts by mass, preferably 3 to 100 parts by mass, more preferably 5 to 80 parts by mass. When the amount is within the range indicated above, whether the carbon black in a sample is a recovered carbon black or a general-purpose carbon black tends to be estimated with high accuracy.

The elastomer composition may contain agents usually used in the tire industry, such as plasticizers, antioxidants, zinc oxide, wax, sulfur, and vulcanization accelerators.

The method for discriminating whether or not a carbon black in the elastomer composition is a recovered carbon black desirably includes: elemental analysis using an electron microscope image of the elastomer composition; comparing a zinc concentration Xc in the field of view including X pieces of primary particles of the carbon black with a zinc concentration Yc in the field of view including Y pieces of primary particles of the carbon black; and comparing the fields of view satisfying a relationship: X > Y.

Specifically, in the comparison of the zinc concentration Xc in the field of view including as many as X pieces of primary particles of the carbon black with the zinc concentration Yc in the field of view including as few as Y pieces of primary particles of the carbon black where the relationship X > Y is satisfied, when the concentration Xc is remarkably higher than the concentration Yc, zinc can be assumed to be localized on or near the surface of the carbon black. Thus, the carbon black can be discriminated as a recovered carbon black. In contrast, when the Xc and the Yc are the same or almost the same, zinc is assumed to be not localized on or near the surface of the carbon black. Thus, the carbon black can be discriminated as a general-purpose carbon black.

In the comparison, when the zinc concentration Xc in the field of view including X pieces of primary particles of the carbon black and the zinc concentration Yc in the field of view including Y pieces of primary particles of the carbon black satisfy the relationship: X > Y, a larger amount of zinc is assumed to be present on or near the surface of the carbon black as compared to the zinc distribution in the entire elastomer composition. This carbon black may probably be estimated as a recovered carbon black. Yet, the Xc and the Yc desirably satisfy the relationship: Xc ≥ 2 × Yc. In this case, it is sufficiently plausible that zinc is localized on or near the surface of the carbon black. Thus, the carbon black can be discriminated with high accuracy as a recovered carbon black.

An exemplary embodiment of the discrimination method of the present invention is described below, but the present invention is not limited to the following method.

Elastomer compositions of Blend 1 and Blend 2 shown in Table 1 are produced.

Blend 1 is an elastomer composition containing a general-purpose carbon black, and Blend 2 is an elastomer composition containing a recovered carbon black.

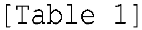

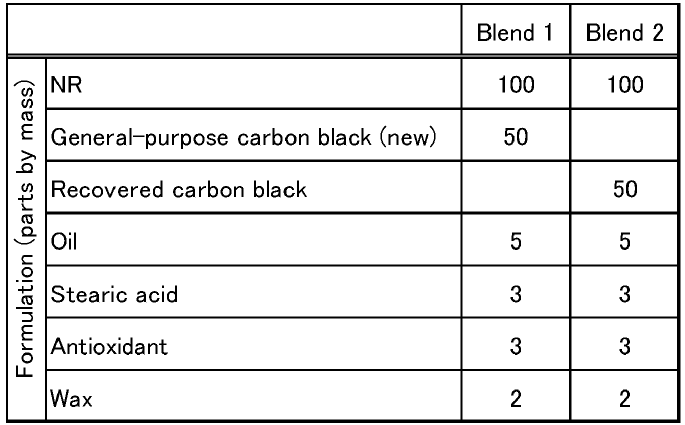

The produced elastomer compositions of Blends 1 and 2 are cooled to -90°C and then cut into thin films with a microtome, whereby samples (elastomer compositions) are produced.

The samples are observed with TEM and images thereof are captured at a magnification at which the primary particle size of the carbon black can be measured. Elemental analysis is performed in the same field of view, and elemental mapping images are obtained.

When the samples are observed in STEM mode with TEM, the carbon black can be observed in the form of white particles (whitish particles) having a primary particle size of 10 to 100 nm as shown in FIG. 1.

In the case of the elastomer composition of Blend 2 containing a recovered carbon black, brighter white particles are seen around the particles which are thought to be the carbon black. This is because parts with a high electron density are bright in a STEM image.

Elemental analysis in the same field of view of the STEM images reveals the following: especially Zn + S (zinc + sulfur) are detected around the carbon black in the elastomer composition of Blend 2 containing a recovered carbon black, while Zn + S (zinc + sulfur) are not detected around the carbon black in the elastomer composition of Blend 1 containing a general-purpose carbon black.

Moreover, in the elemental analysis image of the elastomer composition of Blend 1 containing a general-purpose carbon black, none of Si, Zn, and S is localized in the field of view. In contrast, in the elemental analysis image of the elastomer composition of Blend 2 containing a recovered carbon black, these elements are concentrated around the carbon black and they are colored in the elemental analysis image.

Through the examination of the presence of an inorganic substance such as zinc on or near the surface of the carbon black by elemental analysis using an electron microscope image of an elastomer composition, it is possible to discriminate the carbon black in the sample as a recovered carbon black when an inorganic substance is present or as a general-purpose carbon black when no inorganic substance is present.

As described above, when the region of "on or near the surface of the carbon black" is a region from the surface of the carbon black to the height equal to 200 of the primary particle size of the carbon black, and images in FIG. 1 are examined each in a region from the surfaces of the carbon black particles having a primary particle size of 10 to 100 nm to a height of 2 to 20 nm, brighter white particles are confirmed around (withing 20 nm) the particles which are thought to be the carbon black in the elastomer composition of Blend 2 containing a recovered carbon black. Moreover, Zn, S, etc. are detected by analysis of the bright white particles. Thus, the carbon black can be discriminated as a recovered carbon black.

As described above, in the discrimination method including: elemental analysis using an electron microscope image of the elastomer composition; comparing a zinc concentration Xc in the field of view including X pieces of primary particles of the carbon black with a zinc concentration Yc in the field of view including Y pieces of primary particles of the carbon black; and comparing the fields of view satisfying a relationship: X > Y, for example, when the comparison of the fields of view satisfying a relationship: X > Y is performed by comparing a field of view including 50 pieces or more of primary particles of the carbon black with a field of view including 20 pieces or less of primary particles of the carbon black, and the zinc concentration Xc in the field of view including 50 pieces or more and the zinc concentration Yc in the field of view including 20 pieces or less determined by the elemental analysis satisfy a relationship: Xc ≥ 2 × Yc, it is sufficiently plausible that zinc is localized on or near the surface of the carbon black. Thus, the carbon black can be discriminated with high accuracy as a recovered carbon black.

Accordingly, examining the presence of an inorganic substance on or near a surface of a carbon black in a sample of a carbon black or a sample of an elastomer composition containing a carbon black enables discriminating whether the carbon black is a recovered carbon black or a general-purpose carbon black.

The above-described specific embodiment includes discriminating whether a carbon black is a recovered carbon black or a general-purpose carbon black by comparing an elastomer composition containing a recovered carbon black with an elastomer composition containing a general-purpose carbon black. The technique is also usable to discriminate whether a carbon black in a carbon black sample is a recovered carbon black or a general-purpose carbon black.

Exemplary embodiments of the present invention include the following.

Embodiment 1. A method for discriminating whether or not a carbon black is a recovered carbon black, including
examining the presence of an inorganic substance on or near a surface of the carbon black.

Embodiment 2. The method for discriminating carbon black according to Embodiment 1,
wherein a carbon black is discriminated as a recovered carbon black when an inorganic substance is present on or near a surface of the carbon black.

Embodiment 3. The method for discriminating carbon black according to Embodiment 1 or 2,
wherein the discriminating whether or not a carbon black is a recovered carbon black comprises examining the presence of an inorganic substance on or near a surface of the carbon black in a region from the surface of the carbon black to the height equal to 200 of a primary particle size of the carbon black.

Embodiment 4. The method for discriminating carbon black according to any combination with any one of Embodiments 1 to 3,
wherein the discriminating whether or not a carbon black is a recovered carbon black comprises examining the presence of zinc on or near a surface of the carbon black.

Embodiment 5. The method for discriminating carbon black according to any combination with any one of Embodiments 1 to 4,
wherein the discriminating whether or not a carbon black is a recovered carbon black comprises elemental analysis using an electron microscope image and examining the presence of an inorganic substance on or near a surface of the carbon black.

Embodiment 6. The method for discriminating carbon black according to any combination with any one of Embodiments 1 to 5,
wherein the method is for discriminating whether or not a carbon black in an elastomer composition containing an elastomer component and the carbon black is a recovered carbon black.

Embodiment 7. The method for discriminating carbon black according to Embodiment 6, wherein the method for discriminating whether or not a carbon black is a recovered carbon black includes:
elemental analysis using an electron microscope image of the elastomer composition; and
comparing a zinc concentration Xc in the field of view including X pieces of primary particles of the carbon black with a zinc concentration Yc in the field of view including Y pieces of primary particles of the carbon black, the comparing being performed on the fields of view satisfying a relationship: X > Y.

Embodiment 8. The method for discriminating carbon black according to Embodiment 7,
wherein the relationship to be satisfied is Xc ≥ 2 × Yc.

## Claims

1. A method for discriminating whether or not a carbon black is a recovered carbon black, comprising
examining the presence of an inorganic substance on or near a surface of the carbon black.

2. The method for discriminating carbon black according to claim 1,
wherein a carbon black is discriminated as a recovered carbon black when an inorganic substance is present on or near a surface of the carbon black.

3. The method for discriminating carbon black according to claim 1 or 2,
wherein the discriminating whether or not a carbon black is a recovered carbon black comprises examining the presence of an inorganic substance on or near a surface of the carbon black in a region from the surface of the carbon black to the height equal to 200 of a primary particle size of the carbon black.

4. The method for discriminating carbon black according to any one of claims 1 to 3,
wherein the discriminating whether or not a carbon black is a recovered carbon black comprises examining the presence of zinc on or near a surface of the carbon black.

5. The method for discriminating carbon black according to any one of claims 1 to 4,
wherein the discriminating whether or not a carbon black is a recovered carbon black comprises elemental analysis using an electron microscope image and examining the presence of an inorganic substance on or near a surface of the carbon black.

6. The method for discriminating carbon black according to any one of claims 1 to 5,
wherein the method is for discriminating whether or not a carbon black in an elastomer composition containing an elastomer component and the carbon black is a recovered carbon black.

7. The method for discriminating carbon black according to claim 6,
wherein the method for discriminating whether or not a carbon black is a recovered carbon black comprises:
elemental analysis using an electron microscope image of the elastomer composition; and
comparing a zinc concentration Xc in the field of view including X pieces of primary particles of the carbon black with a zinc concentration Yc in the field of view including Y pieces of primary particles of the carbon black, the comparing being performed on the fields of view satisfying a relationship: X > Y.

8. The method for discriminating carbon black according to claim 7,
wherein the relationship satisfied is Xc ≥ 2 × Yc.
